# EUROPEAN PATENT APPLICATION

(11) **EP 4 659 760 A1**
(43) Date of publication of application: **10.12.2025**
(21) Application number: 24180130.7
(22) Date of filing: 05.06.2024
(51) Int. Cl.: A61K 39/00, A61P 33/02, A61K 31/4168, A61K 31/70, A61K 47/00, C07K 14/00

(54) **THERAPEUTIC IMMUNE INTERVENTION**

(71) Applicant: Helmholtz-Zentrum für Infektionsforschung GmbH, 38124 Braunschweig (DE); Consejo Nacional de Investigaciones Científicas y Tecnológicas, Ciudad Autónoma de Buenos Aires (AR); Universidad de Buenos Aires, C1053ABJ, Ciudad de Buenos Aires (AR); Instituto de Biologia Experimental e Tecnologica, 2781901 Oeiras (PT)
(72) Inventor: SCHULZE, Kai., 38124 Braunschweig (DE); EBENSEN, Thomas., 38124 Braunschweig (DE); GUZMÁN Carlos.A., 38124 Braunschweig (DE); HENNING, Sarah., 38124 Braunschweig (DE); SANCHEZ ALBERTI, Andrés, Ciudad Autónoma de Buenos Aires (1053), Argentina (AR); CERNY, Natacha., Ciudad Autónoma de Buenos Aires (1425), Argentina (AR); BIVONA, Augusto E., Ciudad Autónoma de Buenos Aires (1053), Argentina (AR); FERNÁNDEZ, Marisa M., Ciudad Autónoma de Buenos Aires (1053), Argentina (AR); MALCHIODI, Emilio L., Ciudad Autónoma de Buenos Aires (1053), Argentina (AR); ROLDAO, Antonio., 2781901 Oeiras (PT)
(74) Representative: Kröncke, Rolf

(57) **Abstract**

The present invention relates in a first aspect to a modified, like an acetylated polypeptide representing a modified polypeptide of Traspain or homologs thereof. In addition, the present invention relates to a method for preparing the same and its use in preventive or therapeutic vaccination. Further, a composition is provided comprising the modified, e.g. acetylated polypeptide according to the present invention in combination with an adjuvant, in particular, a cyclic dinucleotide adjuvant. Optionally, an active agent is present in said composition, like Benznidazol or Nifurtimox. In addition, a composition is provided comprising the modified, e.g. acetylated polypeptide according to the present invention or the non-modified polypeptide Traspain combined with an adjuvant and an active agent which is particularly useful in prophylactic and therapeutic vaccination. The modified polypeptide according to the present invention and the respective composition but also the combination of the polypeptide, adjuvant and active agent demonstrated superior properties suitable for use in the field of prevention and immunotherapy of Chagas disease.

## Description

The present invention relates in a first aspect to a modified, like an acetylated polypeptide representing a modified polypeptide of Traspain or homologs thereof. In addition, the present invention relates to a method for preparing the same and its use in preventive or therapeutic vaccination. Further, a composition is provided comprising the modified, e.g. acetylated polypeptide according to the present invention in combination with an adjuvant, in particular, a cyclic dinucleotide adjuvant. Optionally, an active agent is present in said composition, like Benznidazol or Nifurtimox. In addition, a composition is provided comprising the modified, e.g. acetylated polypeptide according to the present invention or the non-modified polypeptide Traspain combined with an adjuvant and an active agent which is particularly useful in prophylactic and therapeutic vaccination. The modified polypeptide according to the present invention and the respective composition but also the combination of the polypeptide, adjuvant and active agent demonstrated superior properties suitable for use in the field of prevention and immunotherapy of e.g. Chagas disease.

### Prior Art

The protozoan parasite *Trypanosoma cruzi* is causing the neglected infectious tropical Chagas disease. Chagas disease affects approximately 8 million people worldwide. Acute infections can proceed to chronicity that can affect 30-40% of infected people with cardiac or gastro enteric disease. Further estimates are that Chagas disease is responsible for 10,600 deaths per year in addition to 97,500 years lived with disability (YLDs). In total, combining years of life lost and YLDs, Chagas disease causes approximately 0.6 million disability adjusted life years (DALYs). Today the main challenge in fighting the parasite remains the development of an effective vaccine to prevent infection and chronic disease progress [Beaumier, C.M. et al. Vaccine, 34, 2996-3000 (2016)]. The vaccine development against Chagas disease faces the challenge that correlates of protection remains to some extent unrevealed as the complexity of a clearing immune response and diverse parasitic immune escape mechanisms hamper the revealing progress. Vaccines as control strategy for Chagas disease not only would have an important impact regarding to public health but also it would be advantageous in economic terms. It has been estimated that Chagas disease in Latin-American countries leads to a loss of about 752,000 working days per year due to premature deaths and it causes an annual 1.2-billion-dollar loss in productivity. In addition to the productivity losses, it is important to consider the economic losses related to healthcare. In this regard it is estimated that the annual global burden would be $627.46 million in healthcare costs. In this context, both prophylactic and therapeutic vaccines against *T. cruzi* would be cost-effective in a broad range of analyzed scenarios taking into account the risk of infection, as well as the price and the efficacy of the vaccine. However, general agreement was found about several immune response pattern characteristics and/or effector mechanisms that seems to be necessary to control *T. cruzi* successfully. Pathogen-specific antibodies belonging to the lgG2a subclass are one of them in murine experimental infection models, as they are associated to a Th1 biased immune response. Moreover, mucosal immunity mediated by IgA antibodies is providing further advantages in the fight against Chagas disease. In fact, the parasite can be transmitted via several mucosal barriers (i.e. conjunctival membrane in the eye, oral route, congenital infection), thus, a first line of defense at the entry site might prevent severe infection progress [Sanchez, L.V. Parasitology, 140, 147-159 (2013)]. However, superior to the humoral immune response is the cellular-mediated one promoted by Th1 effector cells. In this regard, the restricted activation of Th2 cells has a regulating effect [Sanchez Alberti, A. et al. Front Immunol., 11, 128 (2020). CD4+ T-cells producing IFNγ, TNFα or IL-2 are crucial to control the parasite and providing the necessary help for an effective stimulation of CD8+ T-cells. These cells are in turn crucial players in controlling intracellular *T. cruzi* with their cytotoxic effector function. While quantitative increase of pathogen-specific T-lymphocytes is important, also the T-cell quality is of great significance to clear *T. cruzi* infection [Mateus, J. et al. Front Cell Infect Microbiol., 11, 723121 (2021)]. In this regard, multi-functionality of T-cells is associated with a qualitative enhanced immune response and characterized by simultaneous production of the effector cytokines IFNγ, TNFα and IL-2. Moreover, the beneficial influence of IL-17 against *T. cruzi* helps to protect against a severe progress of acute infections [Acevedo, G.R. et al. Front Immunol., 9, 1929 (2018). Thus, it helps to regulate the immune response towards an optimal profile while promoting the prevention of tissue damage. Furthermore, the regulation of the optimal immune response is also dependent on the anti-inflammatory IL-10 as counteracting cytokine that modulate the induced Th1 immune response and prevents pathological side effects to host tissue. Nonetheless, an optimal balance between pro- and anti-inflammatory reactions is absolutely essential as overexpression of IL-10 can lead to non-protective immune responses as well as overexpression of pro-inflammatory cytokines can result in uncontrolled inflammation.

The understanding of Chagas disease, including its pathologies and factors relating to progression, remains limited, and is also challenged by lack of diagnosis, vaccine or an effective treatment. Despite having elapsed more than 120 years since its discovery, Chagas disease remains without an effective treatment, especially for patients with chronic Chagas cardiomyopathy (CCC), the most severe manifestation of the disease, developed by approximately 20-40% of patients and characterized by occurrence of arrhythmias, heart failure and death [Santos EdS, et al. Front. Cell. Infect. Microbiol., 11:765879].

The prolonged duration of chronic Chagas disease is predominantly attributed to the inconspicuous acute infection that often goes unnoticed and a subsequent early asymptomatic progression of the chronic phase. Although the drugs Benznidazole and Nifurtimox are successfully employed during the acute phase, they prove ineffective in reversing chronic infection. Consequently, therapeutic vaccines have emerged as a promising alternative, prompting investigations into their combination with antiparasitic drugs in murine models. A therapeutic vaccine for Chagas disease may improve or even replace the treatment with current drugs (Benznidazole or Nifurtimox) which have several side effects and require long term treatment that frequently leads to therapeutic withdrawal. Finding alternatives for controlling pathology could provide health benefits, socio-economic savings under a wide range of conditions, regardless of the impact of vaccination on reducing clinical progression of the disease.

*T. cruzi* infection presents two main stages: acute and chronic. The 2-3 months acute phase is usually asymptomatic and goes usually unnoticed, being characterized by high level parasitemia. The chronic phase begins when parasitemia decreases and most of infected people remain asymptomatic throughout life. However, after 10-30 years, about 30-40% of infected individuals develop heart or gastro enteric manifestations that affect significantly the quality of life and can lead to death. A major shortcoming is that available drugs for treatment (*e.g*. Nifurtimox and Benznidazole [Bz]) are only effective in the acute or early chronic infection phases. Furthermore, both drugs display a wide range of side effects (*e.g*. central nervous system toxicity, leukopenia), which lead to therapy discontinuation in 10-30% of treated patients.

Traspain, a chimeric antigen tailored to present a multivalent display of domains from key parasitic molecules, has been combined with c-di-AMP as a novel prophylactic strategy [Sanchez Alberti A, et al. NPJ Vaccines, 2017 Apr 10;2:9.]. This formulation proved to be able to prime functional humoral responses and pathogen-specific CD8+ and CD4+ T cells, compatible with a Th1/Th17 bias. However, Traspain has important physical/chemical limitations, affecting its solubility and stability, and in this sense its antigenicity, i.e. capacity to induce a strong CD8+ immune response. Due to aforementioned limitations, Traspain is not very useful as an antigen; it cannot be effectively stabilized in homogeneous solution and its production and purification processes are challenging, rendering impossible to achieve consistent batch to batch protein lots in terms of solubility, antigenicity and functional reproducibility. This problem has blocked the usefulness of the Traspain antigen for clinical application, prompting a search for: (i) antigen improvement via exploring novel compositions and/or formulations, and (ii) process improvement via exploring different purification technologies, towards its industrial applicability. Furthermore, the potential usefulness of Traspain or its derivatives in an immune therapeutic setting has not been previously assessed.

The integration of immunotherapy with antiparasitic drugs, coined as vaccine-linked chemotherapy, emerges as an attractive alternative. This not only complements the action of Bz, but also offers the potential of reducing drug doses, in concentration and times, thereby minimizing the likelihood of adverse effects and patient dropouts.

### Detailed description of the invention

In a first aspect, the present invention relates to a recombinant modified polypeptide comprising a sequence of SEQ ID. No. 1, or that exhibits at least 95% identity to SEQ ID No. 1 or that differs maximally 25 amino acids from the sequence of SEQ ID No. 1 whereby at least one of the cysteine present in SEQ ID. No. 1 is modified by i) mutation including deletion or substitution or ii) by chemical modification of the sulfhydryl group present in the cysteine by forming a S-S linkage between the sulfhydryl group and a S-containing moiety.

In an embodiment, the recombinant modified polypeptide is an acetylated polypeptide comprising a sequence of SEQ ID. No. 1, or that exhibits at least 95% identity to SEQ ID No. 1 or that differs maximally 25 amino acids from the sequence of SEQ ID No. 1 whereby the cysteines present in SEQ ID. No. 1 maintain present, and wherein at least one acetylation of an amino acid cysteine of SEQ ID No. 1 is present whereby the acetyl group is linked to said amino acid cysteine of SEQ ID No. 1 with a S-S linkage between the sulfhydryl group of the cysteine and a S-containing moiety comprising an acetyl group.

In a further aspect, a method for chemical modification, like acetylation of polypeptides defined herein are provided comprising the steps of
i) purification of the denaturated polypeptide;
ii) chemical modification, in particular, acetylation of the polypeptide at the sulfhydryl group of at least one cysteine present in the polypeptide of SEQ ID. No. 1 or that exhibits at least 95% identity to SEQ ID. No. 1 or that differs maximally 25 amino acids from the sequence of SEQ ID. No. 1., preferably with a S-containing moiety comprising an acetyl group;
refolding of the recombinant modified polypeptide and purification thereof.

Furthermore, a composition comprising the modified, e.g. the acetylated polypeptide according to the present invention in combination with an adjuvant is described. Said composition may contain further an active agent, in particular, a therapeutic agent useful in treating Chagas disease. In an aspect, the composition comprises the modified, e.g. the acetylated polypeptide, the adjuvant being a cyclic di-AMP (CDA) and the therapeutic agent is selected from drugs, including drugs with parasitic activity, such as Benznidazol or Nifurtimox.

A further aspect relates to a composition comprising the modified, e.g. the acetylated or non-modified polypeptide according to the present invention, namely, a modified Traspain or the Traspain as such in combination with the adjuvant, like CDA and an active agent like Benznidazol or Nifurtimox. The compositions according to the present invention are useful for preventive or therapeutic vaccination.

The modification, e.g. acetylation of Traspain as described herein, solves several limitations of the Traspain molecule described so far, specifically, the lack of utility, here the industrial applicability, due to limitations of solubility. Namely, the Traspain represents an unstable protein with poor solubility, inferior antigenicity and, in this sense, inadequate immunogenicity and antigenic activity.

### Brief description of the drawings

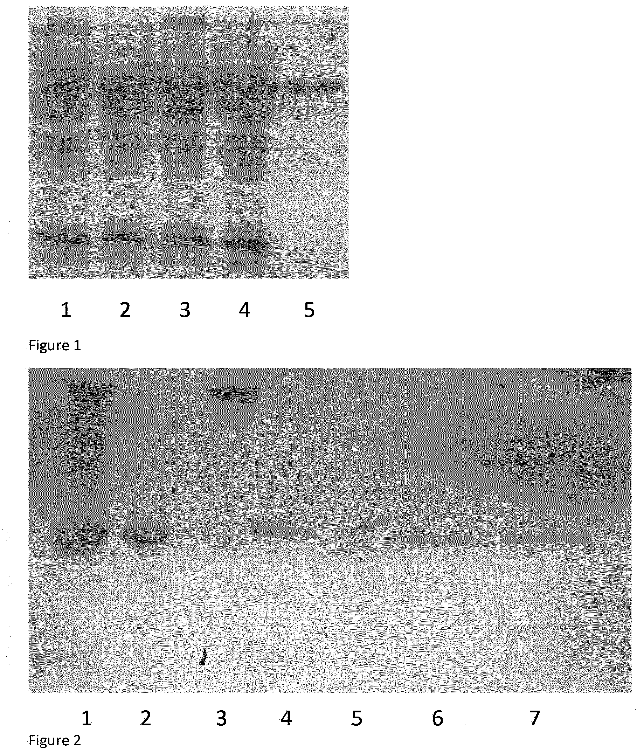

Figure 1. SDS-PAGE of samples collected during the purification process, performed under reducing conditions (i.e. with the addition of 20 mM DTT to sample buffer). Lane 1: Inclusion bodies solubilized at 37°C without DTT; Lane 2: Inclusion bodies solubilized at 37°C with DTT; Lane 3: Inclusion bodies solubilized at RT without DTT; Lane 4: Inclusion bodies solubilized at RT with DTT and Lane 5: Reference Traspain protein.
Figure 2. SDS-PAGE performed under reducing conditions of samples collected during the N-acetyl-cysteine treatment, i.e. N-acetyl-cysteine buffer and DTT-treated Traspain protein were mixed 1:1 and incubated at room temperature (R.T.) for 6 h, protected from light. Lane 1: Traspain before N-acetyl cysteine treatment without DTT; Lane 2: Traspain before N-acetyl cysteine treatment with DTT; Lane 3: Traspain treated with N-acetyl cysteine without DTT precipitate; Lane 4: Traspain treated with N-acetyl cysteine with DTT precipitate; Lane 5: Traspain treated with N-acetyl cysteine without DTT; Lane 6: Traspain treated with N-acetyl cysteine with DTT; Lane 7: Pellet with DTT.

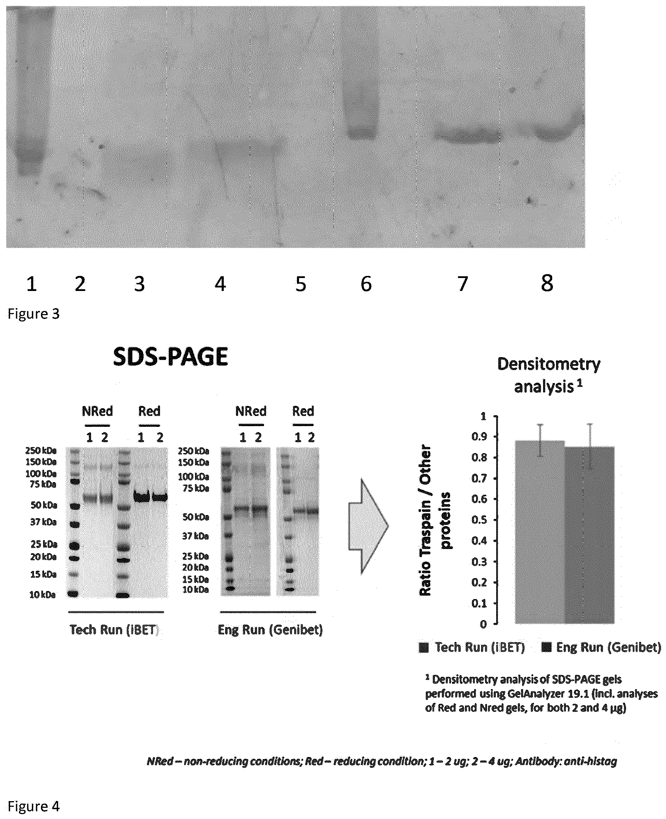

Figure 3. SDS-PAGE of samples collected during the Traspain refolding process by dilution with refolding buffer. Lane 1: Traspain before N-acetyl-cysteine treatment, without DTT; Lane 2: empty; Lane 3: Traspain treated with N-acetyl cysteine without DTT, refolding by dialysis; Lane 4: Traspain treated with N-acetyl cysteine, without DTT, refolding by dilution; Lane 5: empty; Lane 6: Traspain before N-acetyl-cysteine treatment, with DTT; Lane 7: Traspain treated with N-acetyl cysteine, with DTT, refolding by dialysis; Lane 8: Traspain treated with N-acetyl cysteine, with DTT, refolding by dilution.
Figure 4. SDS-PAGE and densitometry analysis of the purified acetylated Traspain protein obtained with the improved process. Left panel: SDS-PAGE of purified acetylated Traspain protein, performed under non-reducing (Nred) and reducing (Red) conditions (1 and 2 lanes correspond to 2 and 4 µg of protein loaded). Right panel: densitometry analysis of SDS-PAGE gels performed using GelAnalyzer 19.1, including Red and Nred conditions for both 2 and 4 µg of protein loaded.

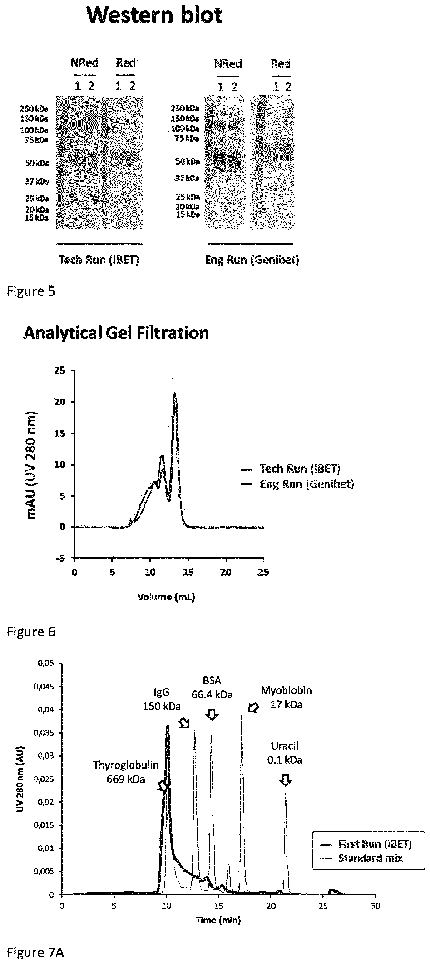

Figure 5. Western blot analysis of the purified acetylated Traspain protein obtained with the improved purification process. SDS-PAGE gels were run under non-reducing (Nred) and reducing (Red) conditions, and revealed using an anti-His antibody.
Figure 6. Analytical size exclusion chromatography (SEC) analysis of the purified acetylated Traspain protein obtained with the improved purification process.

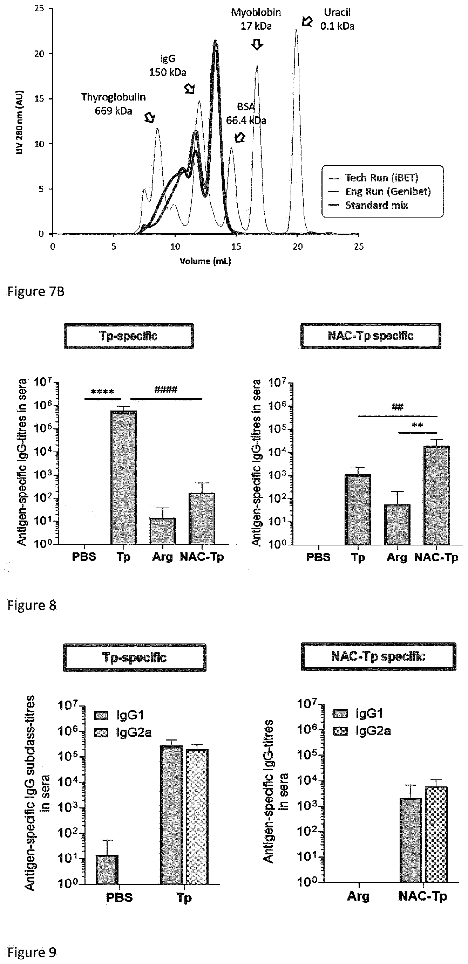

Figure 7. HPLC-SEC of purified non-acetylated Traspain protein obtained with the initial, non-improved purification process (A), and analytical SEC of purified acetylated Traspain protein obtained with the final, improved purification process. The HPLC column used was X Bridge BEH 200Å 3.5 µm (Waters), with mobile phase: 50 mM Tris, 300 mM NaCl, 1 mM EDTA, 2 M urea, 10% glycerol, 5% Sucrose, pH = 8.0, 4°C. The analytical SEC column used was Superdex 200 10/30 GL (Cytiva), with mobile phase: 800 mM Arginine, 50 mM Tris-HCl, 150 mM NaCl, 0.8 mM KCI, 1 mM EDTA, pH = 8.0, RT. Standard mix: mixture of reference compounds of known molecular weights (MW).
Figure 8. Humoral response: IgG antibody response in serum samples. The graphs represent the Traspain-specific (Tp, graph in the left) or the acetylated Traspain-specific (NAC-Tp, graph in the right) Ab response. Both proteins evidenced a higher Ab response against the same protein but a reduced cross-reactivity demonstrating the differences in antigenicity due to the acetylation. Arg: arginine containing buffer used for the NAC-Tp (0.8 M Arginine, 50 mM Tris-HCl, 150 mM NaCl, 0.8 mM KCI, 1 mM EDTA, pH 8.2).
Figure 9. Humoral response: IgG1 and IgG2a subclasses. The graphs represent the Traspain-specific (Tp, graph in the left) or the acetylated Traspain-specific (NAC-Tp, graph in the right) Ab response. Traspain stimulated an enhanced !gG1 compared to IgG2a indicated for a Th2-biased response, in contrast, higher IgG2a titres compared to IgG1 indicated for a shift towards a Th1 response, which was induced by acetylated Traspain.

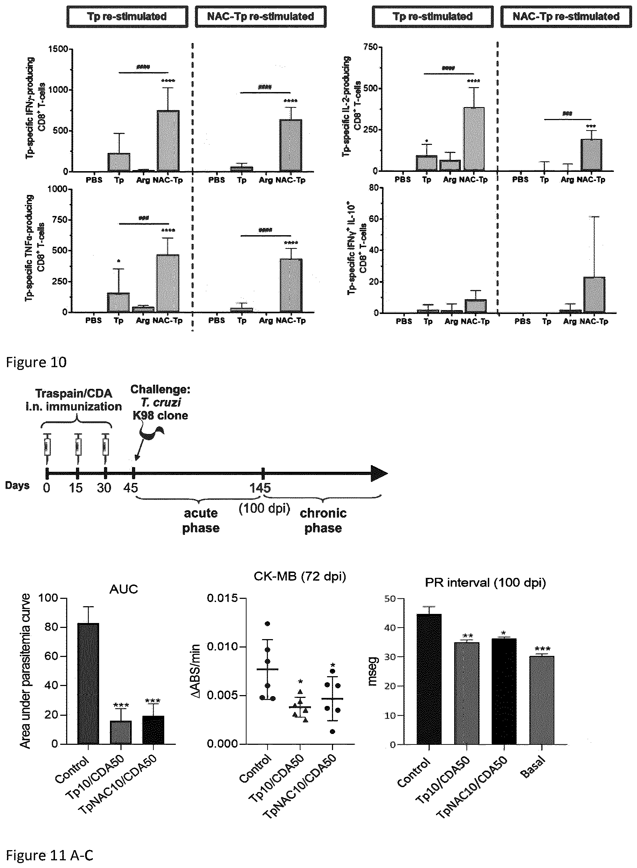

Figure 10. CD8+ T cell stimulation by acetylated Traspain or Traspain in spleen cells stimulated with the homologous and heterologous protein. IFNγ, TNFα and IL-2 producing cells in panels A, B and C. Panel D represents the IFNγ/IL-10 double positive CD8 T cells. Using both antigens for T cell stimulation, acetylated Traspain had the superior ability to stimulate significant enhanced numbers of IFNγ, TNFα and IL-2 producing CD8+ T cells, as well as a higher response for IFNy/IL-10 double positive CD8 T cells.

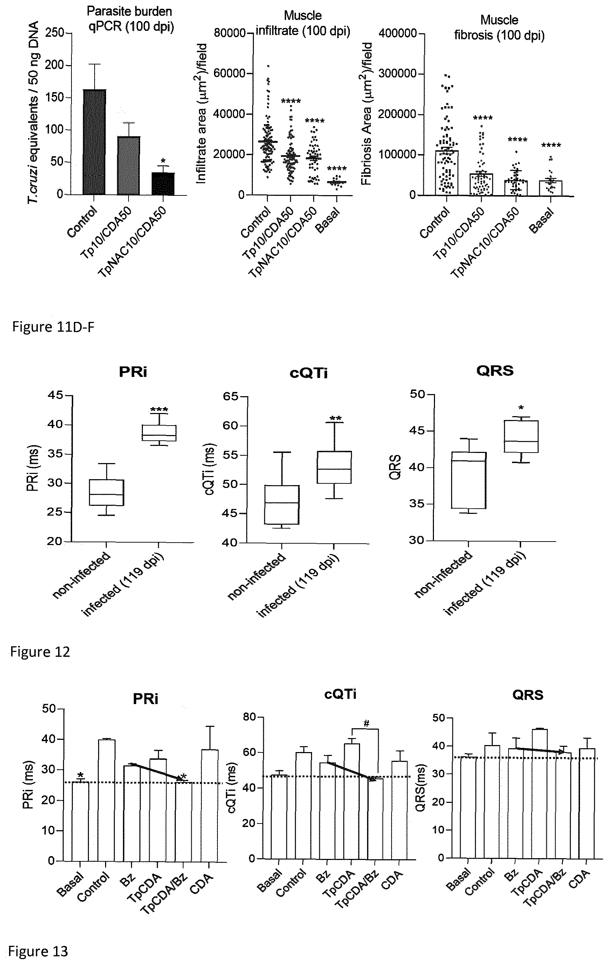

Figure 11. Protection against early chronic infection in vaccinated mice. A) Blood trypomastigotes were counted post-infection in the acute phase (areas under the parasitemia curves); B) ; C) ECG 100 dpi - PR interval; D) Parasite load in muscle at 100 dpi determined by qPCR; E) Inflammatory infiltrate in muscle quantified as nuclei per 5X magnified field, results are expressed as mean; F) Muscle fibrosis quantified as Fibrosis Area (µm2) per 5X magnified field, results are expressed as mean +/- SEM. Asterisks indicate significant differences between groups at. *p <0.05, **p<0.01, *** p<0.001, **** p<0.0001. Student's paired t-test.
Figure 12. Pre-treatment ECG parameters. PRi: PR interval, cQTi: corrected QT interval, QRS: QRS interval. Asterisks indicate significant differences between groups. *p <0.05, **p<0.01, *** p<0.001. Student's paired t-test.
Figure 13. Post-treatment ECG parameters. Vaccine-linked immunotherapy improved all the measured ECG parameters: PR, cQT and QRS intervals. cQTi: corrected QT interval. Results are expressed as Mean +/- SEM. Asterisks "*" indicate significant differences with respect to control group, * p<0.05; "#" indicates significant differences between the specified groups, # p<0.05. One-way ANOVA+ Tukey's post-test.

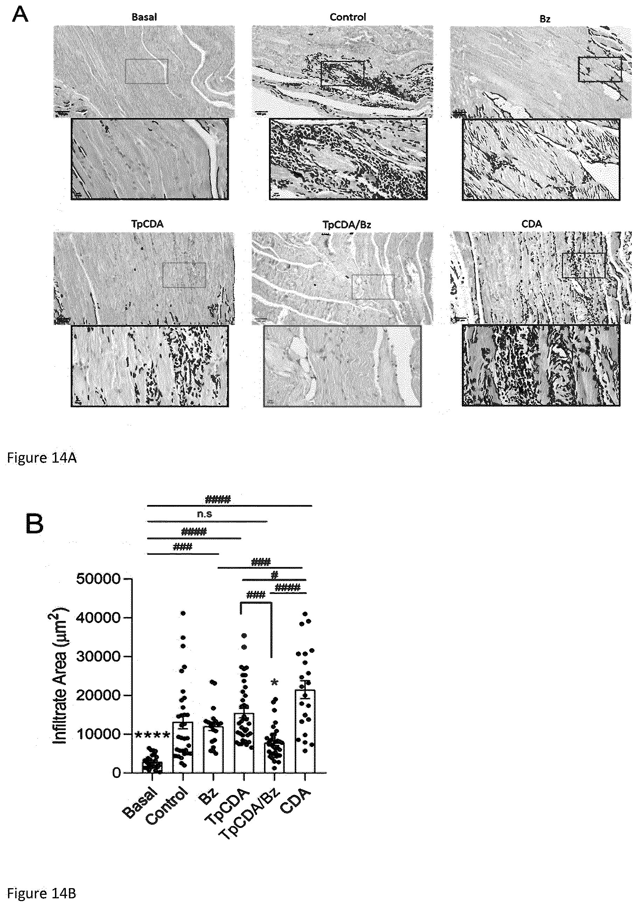

Figure 14. Inflammatory infiltrate in skeletal muscle. A) Representative photomicrographs of sections stained with haematoxylin-eosin. Total magnification: 5X and 20X (red borders). B) Inflammatory infiltrate in skeletal muscle quantified as the Infiltrate Area (µm²) per 5X magnified field Results are expressed as Mean +/- SEM. Asterisks "*" indicate significant differences with respect to control group, * p<0.05, **** p< 0.0001; "#" indicates significant differences between the specified groups, # p<0.05, ### p<0.001, #### p<0.0001. n.s. not significant. One-way ANOVA + Tukeys's post-test.

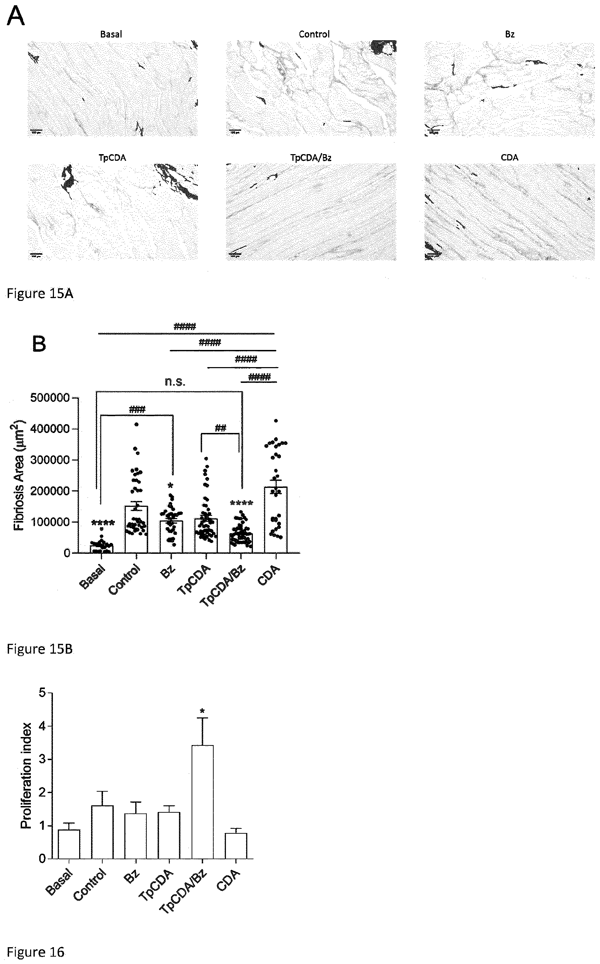

Figure 15. Fibrosis in skeletal muscle. A) Representative photomicrographs of sections stained with Picrosirius Red. Total magnification: 5X. B) Fibrosis in skeletal muscle quantified as Fibrosis Area (µm²) per 5X magnified field. Results are expressed as Mean +/- SEM. Asterisks "*" indicate significant differences with respect to control group, * p<0.05, **** p< 0.0001; "#" indicates significant differences between the specified groups, ## p<0.01, ### p<0.001, #### p<0.0001. n.s. not significant; One-way ANOVA + Tukey's post-test.
Figure 16. Traspain-specific proliferation. Splenocytes from different groups were stimulated with recombinant Traspain and antigen-specific proliferation was determined by the incorporation of ³H-thymidine. Asterisks indicate significant differences compared to the control group. *p <0.05. One-way ANOVA, Dunnett's post-test.

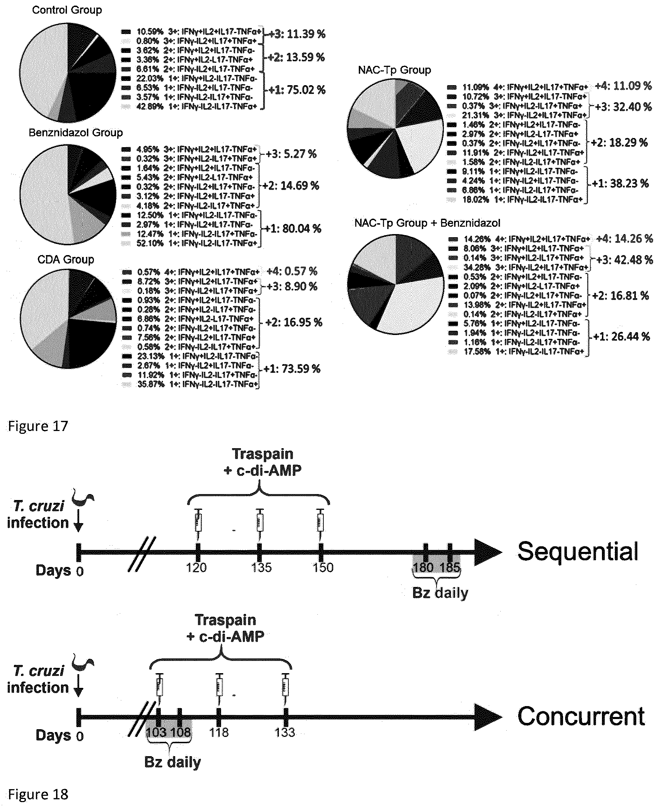

Figure 17. Proportion of polyfunctional T cells. The number of simultaneous functions is expressed in the range of +4 to +1.
Figure 18. Sequential and concurrent therapeutic vaccine-linked chemotherapy schemes. Infected animals were either vaccinated and subsequently received a course of Benzmidazol (upper graphic) or were concurrently treated with Benzmidazol and received the 3 doses of the therapeutic vaccine (lower graphic).

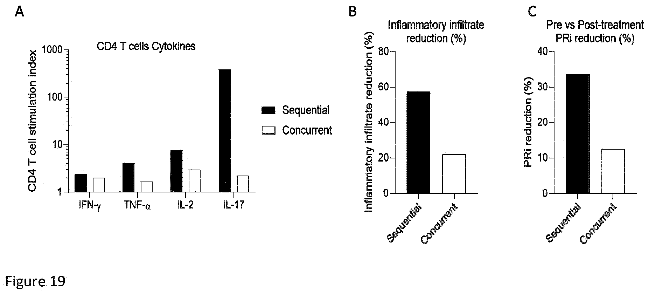

Figure 19. Sequential and concurrent therapeutic vaccine-linked chemotherapy. A) CD4 T cell cytokine production expressed as a ratio with respect to a non-treated control group. B) Inflammatory infiltrate reduction (%) as compared to a control group. C) Electrocardiogram PR interval reduction (%) as compared to a control group.

### Detailed description of the present invention

The present invention comprises a novel vaccine antigen for prevention or treatment of Chagas disease, obtained by a novel method that comprises one step of modification, e.g. acetylation, resulting in a different antigen, however suitable for industrial applicability due to their physical and chemical nature that favors the stability, consistency, reproducibility and scalability of the antigen as well as a superior antigen with higher Th1/CD8+ T cell immunogenicity, rendering an antigen with a higher functional activity.

Namely, in a first aspect, the present invention relates to a recombinant modified polypeptide comprising a sequence of SEQ ID. No. 1, or that exhibits at least 95 % identity to SEQ ID No. 1 or that differs maximally 25 amino acids from the sequence of SEQ ID No. 1 whereby at least one of the cysteine present in SEQ ID. No. 1 is modified by i) mutation including deletion or substitution and/or ii) by chemical modification of the sulfhydryl group present in the cysteine by forming a S-S linkage between the sulfhydryl group and a S-containing moiety.

For example, the recombinant modified polypeptide is an acetylated polypeptide comprising a sequence of SEQ ID. No. 1, or that exhibits at least 95% identity to SEQ ID No. 1 or that differs maximally 25 amino acids from the sequence of SEQ ID No. 1 whereby the cysteines present in SEQ ID. No. 1 maintain present, and wherein at least one acetylation of an amino acid cysteine of SEQ ID No. 1 is present whereby the acetyl group is linked to said amino acid cysteine of SEQ ID No. 1 with a S-S linkage between said cysteine and a S-containing moiety comprising an acetyl group.

The term "modification" or the term "modified" refers to polypeptide wherein at least one amino acid has been modified as described. A modification includes a mutation which may be a deletion or substitution of an amino acid, a cysteine, present in the polypeptide. Alternatively or in addition, the modification refers to a chemical modification of the cysteine present in the sequence. The modification takes place at the sulfhydryl group of the cysteine forming a S-S linkage between the sulfhydryl group a S-containing moiety. A typical example of chemical modification include the acetylation of the cysteine.

In this connection, the term "acetylated polypeptide" refers to an acetylated Traspain or homologs thereof. Traspain is a synthetic antigen encompassing domains of three different proteins of the protozoan parasite *Trypanosoma cruzi.*

The modified, e.g. the acetylated Traspain is different to the unmodified Traspain in terms of physical and chemical properties, resulting in an antigen suitable for industrial scale-up, with a different antigenicity, superior immunogenicity and immune-prophylactic and -therapeutic capacity compared to the non-modified Traspain, useful in the prevention and treatment of Chagas disease.

Modified, e.g. Acetylated Traspain contains the amino acid sequences of domains of three different proteins of the parasite *T. cruzi,* obtained by genetic engineering of SEQ ID No. 1, and obtained using a method that encompasses the modification, e.g. acetylation in one of the production steps. The novel antigen is not only suitable for industrial scale-up due to their physical and chemical properties, it is in turn able to stimulate immune response and protection leading to the prevention of the damage caused by the pathogen with a superior capacity to non-modified Traspain and it is also suitable for the combination with other existing treatments, reaching a superior effect in more advanced Chagas disease, as compared to the conventional chemical treatment as demonstrated below.

The term "recombinant modified polypeptide" and "acetylated polypeptide" as used herein comprises, unless otherwise indicated, also polypeptides exhibiting at least 95% identity to SEQ ID No. 1 or differing maximally 25 amino acids from the SEQ ID No. 1 whereby the cysteines present in SEQ ID No. 1 maintain present. That is, the variant polypeptides also termed homologs thereof, can be generated using genetic engineering, e.g. by insertion, substitution, deletion or a combination thereof. In the protein sequence of the invention, the changes can be conservative or non-conservative. As said, the cysteines are not changed but maintain as they are in the sequence. It is possible to insert or substitute further cysteine by genetic engineering. The polypeptide exhibits at least 95% identity like at least 97% identity or at least 99% amino acid sequence identity with SEQ ID No. 1. In addition, the polypeptide may differ maximally 25 amino acids from SEQ ID No. 1, like maximally 15 amino acids, e.g. maximally 10 amino acids from the sequence of SEQ ID No. 1.

In addition, the polypeptide according to the present invention represents a recombinant polypeptide composed of the amino acid sequences of domains of the three different proteins of *T. cruzi.* Based on the system used for the production of the recombinant polypeptide, further post-translational modifications may be present.

As said, in an embodiment of the present invention the acetylated polypeptide is characterized in having an acyl group linked to an amino acid residue cysteine of SEQ ID No. 1. The acetyl group is linked via a S-S-linkage between the cysteine present in SEQ ID. No. 1 and a S-containing moiety further comprising the acetyl group.

A typical example of the S-containing moiety comprising the acetyl group allowing S-S-linkage with the cysteine present in the polypeptide of SEQ ID No. 1 or homologs thereof is N-acetyl-cysteine. Of course, other possible acetyl containing and S-containing are possible which enable forming a S-S-linkage with cysteine amino acid residues.

In an embodiment, the acetylated polypeptide according to the present invention is a polypeptide with at least two acetylation of amino acid residue cysteines of SEQ ID No. 1 present. That is, the acetyl group is present with at least two cysteines of SEQ ID No. 1 or the homologs thereof. For example, at least three acetylation's, like at least four, at least five, at least six, at least seven, at least eight, at least nine or all cysteines present in SEQ ID No. 1 are acetylated.

Further, the modification by mutation may be achieved by genetic engineering. The skilled person is well aware of suitable methods and expression systems allowing the production of the recombinant modified polypeptide.

In addition, the modified, e.g. the acetylated polypeptide according to the present invention may comprises a protein tag. As used herein, the term "protein tag" refers to a moiety, namely a peptide sequence genetically grafted onto a recombinant polypeptide. Tags are attached to the polypeptide for various purposes. Typically, the protein tag is present either at the C-Terminus or N-Terminus of the polypeptide. Often, the protein tag is used for purification of the recombinant polypeptide, e.g. the protein tag is an affinity tag e. g. chitin binding protein, Maltose binding protein, Strep tag and glutathione-S-transferase. In a preferred embodiment, the protein tag is a poly His tag. In another embodiment of the present invention, no tag or a cleavable tag is present.

In an embodiment, the acetylated polypeptide according to the present invention is a polypeptide of SEQ ID No. 1 with at least one acetyl cysteine bound to a cysteine present in the sequence of SEQ ID No. 1.

The modified, e.g. the acetylated polypeptide demonstrates improved solubility compared to the non-modified polypeptide, the Traspain. In an embodiment, the acetylated polypeptide has a solubility in water of at least 1 mg/ml, like at least 2 mg/ml, e.g. at least 4 mg/ml.

In a further aspect, the present invention relates to a method for chemical modification of recombinant polypeptides according to the present invention comprising the steps of
i) purification of the denaturated polypeptide;
ii) chemical modification, in particular, acetylation of the polypeptide at the sulfhydryl group of at least one cysteine present in the polypeptide of SEQ ID. No. 1 or that exhibits at least 95% identity to SEQ ID. No. 1 or that differs maximally 25 amino acids from the sequence of SEQ ID. No. 1., preferably with a S-containing moiety comprising an acetyl group;
iii) refolding of the recombinant modified polypeptide and purification thereof.

Particularly, the present invention relates to a method for acetylation of polypeptides according to the present invention, comprising the steps of
i) purification of the denaturated polypeptide;
ii) acetylation of the polypeptide with a S-containing moiety comprising an acetyl group;
iii) refolding of the acetylated polypeptide and purification thereof.

As detailed below, the acetylation requires certain steps to obtain the purified acetylated Traspain or homolog thereof according to the present invention. In an embodiment, the S-containing moiety comprising an acetyl group is N-acetylcysteine.

Moreover, the present invention relates to the modified, e.g. the acetylated polypeptide according to the present invention obtainable with the method according to the present invention as detailed herein.

The subject matter also comprises the formulations of modified, e.g. acetylated Traspain, suitable both for parenteral and mucosal administration, including immunostimulatory compounds, adjuvants, and excipients, specifically -but not limited to-"cyclic-di nucleotides, more specifically "cyclic di-adenosine monophosphate", also called c-di-AMP or CDA. This novel antigen can be administered in more complex adjuvant formulations, in combination with other biological response modifiers and more traditional adjuvants like Alum to generate preventive and therapeutic vaccine formulations.

The invention also comprises the formulation of modified, e.g. acetylated Traspain with conventional or novel adjuvants suitable for mucosal and parenteral routes, designed to optimize the preventive and therapeutic effect of the resulting formulation, as well as the method of treatment with the product alone, modified, e.g. acetylated or not, or combined with other conventional therapeutic agents, including but not restricted to Benznidazol with the aim of delaying or blocking the progression of the Chagas disease to severe cardiopathy.

Namely, in a further aspect, the present invention relates to a composition comprising i) a modified, e.g. an acetylated polypeptide according to the present invention, and ii) an adjuvant and, optionally, iii) an active agent, in particular, a therapeutic agent, like Benznidazol or Nifurtimox.

In addition, the composition according to the present invention may be a composition comprising i) a modified, e.g. an acetylated or non-modified polypeptide as defined herein, ii) an adjuvant and iii) an active agent, in particular, a therapeutic agent, like Benznidazol or Nifurtimox.

Further, the present invention relates to a composition for use in therapeutic vaccination comprising
i) a non-modified polypeptide comprising a sequence of SEQ ID. No. 1 as defined herein;
ii) an adjuvant and optionally,
iii) an active agent, in particular, a therapeutic agent, like Benznidazol or Nifurtimox

In the compositions according to the present invention, the adjuvant may be selected from suitable adjuvants known to the skilled person. For example, the adjuvant is a cyclic-di-nucleotide described in the art. In an embodiment, the adjuvant is cyclic di-AMP. As mentioned above, the composition according to the present invention may optionally be administered in combination with one or more anti-parasitic drug in order to obtain the desired synergistic therapeutic effect. The combination results in an unpredicted synergistic effect, superior to any of these individual approaches applied separately. The therapeutic effect goes beyond a simple potential additive effect resulting from each of the separated therapies/interventions and also goes beyond what would have been expected by the combined effect of both products. In fact, until now the chemotherapy is described as being effective only during the early stage of infection and now
the present invention allows a qualitative improvement of the resulting treatment evidencing efficacy in more advanced Chagas disease. Both treatments are suitable for their simultaneous administration as well as for sequential treatment, an unexpected and surprising effect of the therapeutic combination. In an embodiment of the present invention, the sequential treatment is conducted. For example, the sequential treatment comprises the administration of the vaccine first followed by the administration of the drug. In an embodiment, the present invention refers to the therapeutic vaccination and treatment of infected individuals.

One specific embodiment of the present invention comprises the therapeutic potential of native or modified, e.g. acetylated Traspain formulations in a sequential or simultaneous administration with the anti-parasitic drug Benznidazole (Bz) in a combination of native or modified, e.g. acetylated Traspain treatment and chemotherapy that results in a synergistic effect of the combination in terms of improved prevention of target tissue damage and the recovery of the heart normal electroconductive activity. This embodiment of the present invention also addresses the use of lower dosages of Benznidazol, thereby improving both tolerance and adherence to the medication, two mayor bottlenecks in the conventional antiparasitic therapy.

The transformed modified, e.g. acetylated Traspain remain similar in primary sequence compared to the Traspain antigen, encompassing the N-terminal domain of Cruzipain (Cz)-the major cysteine protease of *T. cruzi-,* the central region of amastigote surface protein 2 (ASP2)-an antigen expressed exclusively during the intracellular stage-, and a long alfa chain sequence of an inactive transialidase (iTS)-a major antigen and virulence factor.

For example, the acetylated Traspain can be obtained by a 8 step novel purification method that includes one step of acetylation, characterized by:
**Step 1. Cell disruption and inclusion bodies washing:** cell lysis with 50 mM Tris-HCl, 100 mM NaCl, 0.1 mM EDTA, 5% Glycerol, pH 8.0 at RT, followed by three washing steps with 50 mM Tris-HCl, 100 mM NaCl, 0.1 mM EDTA, 5 % glycerol, 1% Triton X-100, pH 8.0 at RT (washing step 1), 50 mM Tris-HCl, 100 mM NaCl, 0.1 mM EDTA, 5% Glycerol, pH 8.0 at RT (washing step 2), and 50 mM Tris-HCl, 100 mM NaCl, 0.1 mM EDTA, 5% Glycerol, 4 M urea, pH 8.0 at RT (washing step 3).
**Step 2. Solubilization of inclusion bodies**: solubilization of inclusion bodies with 50 mM Tris, 6 M guanidinium chloride, 100 mM DTT, pH 8 at RT, followed by dilution 1:10 in 50 mM Tris, 6 M guanidinium chloride, pH 8 at RT.
**Step 3. Traspain purification via affinity chromatography**: immobilized metal ion affinity chromatography (IMAC), using as equilibration buffer 50 mM Tris, 6 M guanidinium chloride, 10 mM DTT at pH 8.0 at RT, as washing buffer 50 mM Tris, 6 M guanidinium chloride, 10 mM DTT at pH 6.3 at RT, and as elution buffer 50 mM Tris, 6 M guanidinium chloride, 10 mM DTT at pH 4.5 at RT.
**Step 4. Traspain treatment with N-acetylcysteine (NAC)**: IMAC eluted pool was treated with NAC 300 mM and DTT 100 mM, and incubated for 3 h at RT.
**Step 5. Traspain refolding via dropwise dilution**: dilution of NAC treated Traspain 1:20 to refolding buffer (800 mM Arginine, 50 mM Tris-HCl, 150 mM NaCl, 0.8 mM KCI, 1 mM EDTA, pH 8.2 at RT), followed by filtration 0.2 µm, concentration 20x in 800 mM arginine, 50 mM Tris-HCl, 150 mM NaCl, 0.8 mM KCI, 1 mM EDTA, pH 8.2 at RT, and dilution 1:20 to refolding buffer.
**Step 6. Traspain concentration via tangential flow filtration (TFF):** concentration of refolded Traspain via TFF in refolding buffer (800 mM arginine, 50 mM Tris-HCl, 150 mM NaCl, 0.8 mM KCI, 1 mM EDTA, pH 8.2 at RT).
**Step 7. Traspain purification via size exclusion chromatography:** Traspain was purified using Superdex 200 50/100 column, using as buffer 800 mM arginine, 50 mM Tris-HCl, 150 mM NaCl, 0.8 mM KCI, 1 mM EDTA, pH 8.2 at RT.
**Step 8. Endotoxins removal by ion exchange chromatography:** endotoxins were removed from purified Traspain solution using Sartobind STIC PA as anion exchanger and 800 mM arginine, 50 mM Tris-HCl, 150 mM NaCl, 0.8 mM KCI, 1 mM EDTA, pH 8.2 at RT as buffer.

In an embodiment, the addition of conventional adjuvants and/or biological response modifiers c-di-nucleotides, like c-di-AMP, are used to ensure the quality of the immune response required for parasite clearance by the induction of a strong, fast and high avidity antibody response and the appropriate cellular immune response pattern, thereby promoting enhanced binding, neutralization or killing activity.

The subject matter of the present invention also comprises the resulting formulations of Traspain as defined herein being a modified or non-modified polypeptide according to the present invention in combination with c-di-AMP and with other potential immune stimulators to improve the effect of c-di-AMP, allowing dose splitting or adding complementary properties to the resulting formulation, in particular in terms of effector functions responsible for the clearance of *T. cruzi,* as well as enhancing the innate immunity activation with agonists of pattern recognition receptors, such as TLRs. The present invention also includes the method of preventing Chagas disease onset or progression, and/or disease severity, preferably by the nasal or mucosal route, in a dose range capable of eliciting an effective antibody and/or cellular immune response.

That is, the composition may be also present in at least two separate components wherein the first component comprises the modified, e.g. the acetylated polypeptide and the adjuvant, and the second component comprises an active agent.

One specific embodiment of the present invention comprises the formulations of modified, e.g. acetylated Traspain and other proteins from *T. cruzi.* In a preferred embodiment, the formulations will comprise a c-di-nucleotide, like c-di-AMP, in a range of concentrations allowing a stronger and well-modulated immune response according to the current pharmacological studies, the range comprise the concentrations from 0.020 mg/mL up to 0.300 mg/mL of c-di-AMP in phosphate buffered saline solution (PBS) or other compatible buffer.

In a preferred embodiment, the formulations of the present invention can be administered by mucosal route (e.g. intranasal, sublingual, intrapulmonic, oral) or alternatively by parenteral route (e.g. intramuscular, subcutaneous, intradermal), alone or as part of a formulation with alum or other depot adjuvants, formulated with different adjuvants or just mixed at the moment of product administration.

In a preferred embodiment of the present application, the formulation will be administered in accelerated schedules of immunization considering the application every week, every 15 days or appropriately adjusted to the requirement, as well as in long-term schedules tailored to time the boosting with the contraction phase of the germinal center formation to foster hypersomatic mutation and affinity maturation, as well as in prime-pull protocols.

The method according to the present invention for preventive or therapeutic vaccination against Chagas disease comprises the administration of the compositions or the modified, e.g. The acetylated polypeptide according to the present invention for example mucosally, in particular, by intranasal, oral, sublingual, inhalation, or systemically, like intramuscular, subcutaneous, intradermal or by using patches, spray devices, droppers.

The present invention will be described further by way of examples without limiting the same thereto.

### Examples

### Example 1. Acetylated Traspain production and purification method

A novel vaccine antigen (acetylated Traspain as an example of modified Traspain), has been produced using a novel method comprising 8 steps, rendering a superior antigen in terms of stability, antigenicity, immunogenicity and functional activity as a protective vaccine antigen for prophylactic or therapeutic antigen. An antigen with superior stability in mucosal and parenteral formulations has been obtained as shown by their higher stability in solution allowing their industrial use and manufacturing compared to the non-acetylated protein. The new antigen has superior properties and is suitable for their use in the field of prevention and immunotherapy of Chagas disease.

The acetylated Traspain was obtained by an 8 steps novel purification method that includes a final step of acetylation, characterized by:
**Step 1. Cell disruption and inclusion bodies washing:** cell lysis with 50 mM Tris-HCl, 100 mM NaCl, 0.1 mM EDTA, 5% glycerol, pH 8.0 at RT, followed by three washing steps with 50 mM Tris-HCl, 100 mM NaCl, 0.1 mM EDTA, 5% glycerol, 1% Triton X-100, pH 8.0 at RT (washing step 1), 50 mM Tris-HCl, 100 mM NaCl, 0.1 mM EDTA, 5% glycerol, pH 8.0 at RT (washing step 2), and 50 mM Tris-HCl, 100 mM NaCl, 0.1 mM EDTA, 5% glycerol, 4 M urea, pH 8.0 at RT(washing step 3).
**Step 2. Solubilization of inclusion bodies:** solubilization of inclusion bodies with 50 mM Tris, 6 M guanidinium chloride, 100 mM DTT, pH 8 at RT, followed by dilution 1:10 in 50 mM Tris, 6 M guanidinium chloride, pH 8 at RT.
**Step 3. Traspain purification via affinity chromatography:** immobilized metal ion affinity chromatography (IMAC), using as equilibration buffer 50 mM Tris, 6 M guanidinium chloride, 10 mM DTT at pH 8.0 at RT, as washing buffer 50 mM Tris, 6 M guanidinium chloride, 10 mM DTT at pH 6.3 at RT, and as elution buffer 50 mM Tris, 6 M guanidinium chloride, 10 mM DTT at pH 4.5 at RT.
**Step 4. Traspain treatment with N-acetylcysteine (NAC):** IMAC eluted pool was treated with NAC 300 mM and DTT 100 mM, and incubated for 3 h at RT.
**Step 5. Traspain refolding via dropwise dilution:** dilution of NAC treated Traspain 1:20 to refolding buffer (800 mM arginine, 50 mM Tris-HCl, 150 mM NaCl, 0.8 mM KCI, 1 mM EDTA, pH 8.2 at RT), followed by filtration 0.2 µm, concentration 20x in 800 mM arginine, 50 mM Tris-HCl, 150 mM NaCl, 0.8 mM KCI, 1 mM EDTA, pH 8.2 at RT, and dilution 1:20 to refolding buffer.
**Step 6. Traspain concentration via tangential flow filtration (TFF):** concentration of refolded Traspain via TFF in refolding buffer (800 mM arginine, 50 mM Tris-HCl, 150 mM NaCl, 0.8 mM KCI, 1 mM EDTA, pH 8.2 at RT).
**Step 7. Traspain purification via size exclusion chromatography:** Traspain was purified using Superdex 200 50/100 column, using as buffer 800 mM arginine, 50 mM Tris-HCl, 150 mM NaCl, 0.8 mM KCI, 1 mM EDTA, pH 8.2 at RT.
**Step 8. Endotoxins removal by ion exchange chromatography:** endotoxins were removed from purified Traspain solution using Sartobind STIC PA as anion exchanger and 800 mM Arginine, 50 mM Tris-HCl, 150 mM NaCl, 0.8 mM KCI, 1 mM EDTA, pH 8.2 at RT as buffer.

The figure 1 presents the assessment by polyacrylamide gel electrophoresis (SDS-PAGE) of the solubilized Traspain protein during the purification process, namely the inclusion bodies solubilization step (Step 2). Inclusion bodies were solubilized at 37°C and also at room temperature, with or without of DTT. In this purification step the purity of the protein is significantly increased. The figure 2 presents the assessment by SDS-PAGE of the solubilized Traspain protein before and after the treatment with N-acetylcysteine (at 300 mM), with DTT (at 100 mM) or without DTT. Figure 3 presents the assessment by SDS-PAGE of the Traspain refolding process via dilution or dialysis, before and after the treatment with N-acetylcysteine, with or without DTT. The resulting protein achieved a >95% purity, and it is soluble in their final buffer rendering a homogeneous solution in comparison to what observed with Traspain, namely more stable and useful for industrial scale up and applicability in formulation and vaccination studies. The figure 4 presents the assessment by SDS-PAGE of the purified and solubilized acetylated Traspain protein obtained with the improved purification process during the technical and engineering manufacturing runs, as well as the densitometry analysis of the resulting bands. The figure 5 presents the assessment by Western blot of the purified, solubilized acetylated Traspain protein obtained with the improved purification process during the technical and engineering manufacturing runs. The figure 6 presents the assessment by analytical gel filtration of the purified, solubilized acetylated Traspain protein obtained with the improved purification process during the technical and engineering runs, showing the monomer and the main oligomers. The figure 7 presents the assessment of the purified non-acetylated Traspain protein obtained with the initial, non-improved purification process (by HPLC-SEC), and of purified acetylated Traspain protein obtained with the final, improved purification process (by analytical SEC).

### Example 2. Acetylated Traspain stimulated a preferred Th1 immune response with a stronger CD8 T cell response.

Acetylation is recognized as a modification according to the present invention that can dramatically change the structure and function of a protein. However, the impact of acetylation is associated to a change in conformation and physical and chemical characteristics, it is not obvious that this process may render an antigen with more attractive characteristics, indeed it might be expected the opposite as a result of structural changes. The method of acetylation, the level of acetylation, the protein that is being transformed and the specific epitopes that are affected are some of the variables that interact in the resulting properties of the transformed protein. In this sense we decided to explore the antigenicity and immunogenicity of acetylated Traspain after confirming that the specific process of production render a stable and homogeneous subunit antigen in solution, which display at least non-inferiority functional properties as vaccine antigen.

To characterize the new immunological properties of the acetylation on Traspain, four groups of mice received three times in a 14 days interval 10 µg antigen combined with 50 µg of CDA via intranasal route. Mice were immunized with Traspain dissolved in PBS or acetylated Traspain dissolved in arginine buffer. PBS or arginine buffer (Arg) were administered in control groups.

Safety and tolerability was monitored over the whole course of vaccination, characterized by changes in the locomotor and exploratory behavior, posture, fur appearance and body weight. Both vaccine formulations were well tolerated, although mice showed a slight and reversible weight reduction that was lower in the group immunized with the acetylated Traspain evidencing a superior safety profile.

The analysis of the antibody (Ab) response evidenced that the protein antigenicity was different when both Traspain and acetylated Traspain were compared. Both protein antigens evidenced a capacity to trigger high Ab responses against the homologous protein, but there was a reduced cross-reactivity, thereby demonstrating differences in the antigenicity due to the acetylation. This suggested that the acetylation has created conformational changes resulting in new epitopes that changed the B-cell antigenicity of the modified protein, making impossible to predict if this could in turn lead to changes in the capacity of the acetylated protein to promote protective responses (Figure 8).

For a detailed insight into the immune response pattern stimulated by vaccination, the subclass antibody distribution was evaluated. In this regard, the stimulation of enhanced IgG1 compared to IgG2a indicated for a Th2-biased response, which was elicited by Traspain, as shown in Figure 9. In contrast, higher IgG2a titers compared to IgG1 indicated for a shift towards a Th1 response, which was induced by acetylated Traspain. This is indeed a more favorable immune response pattern predicted to confer a superior protection against the parasite. Moreover, both proteins induced similar mucosal IgA responses as detected in lung secretions assessed against the homologous protein.

The analysis of the CD8+ T-cell subset is of great relevance as the CD8+ T cell responses have been correlated to protective responses against *T. cruzi.* The results revealed that the acetylated Traspain had the superior ability to stimulate significant enhanced numbers of IFNγ, TNFα and IL-2 producing cells, regardless that cells were re-stimulated with homologous or heterologous protein (Figure 10). Moreover, the acetylated Traspain induced a clear trend for the stimulation of antigen-specific CD8+ T-cells being double-positive for IFNγ and IL-10. This cellular subpopulation seems to play a significant role in protection by limiting parasite burden and protecting against myocarditis [Ester Roffê et al. Immunol. 2012; 188(2): 649-660. doi:10.4049/jimmunol.1003845]. The acetylated Traspain was also superior in inducing elevated numbers of multifunctional CD8+ T-cells. It is also important to highlight the superior capacity of acetylated Traspain to promote production of IL-9, as determined by Th multiplex assay. IL-9 is expected to play a protective role in immune pathogenesis by controlling cardiac fibrosis and pro-inflammatory cytokine production (Nadjania Saraiva de Lira Silva et al. Front Cell Infect Microbiol. 2021 Oct 15;11:756521. doi: 10.3389/fcimb.2021.756521). Thus, these results support the relevance of acetylated Traspain in the prevention and potential for the immunotherapy of Chagas Infection.

### Example 3. Prophylactic effect of Acetylated Traspain

Comparative studies of acetylated Traspain and Traspain were performed to assert its non-inferiority in terms of conferring protection against early chronic disease in vaccinated animals (Figure 11). Thus, to evaluate the ability of the vaccine to prevent chronic stage-associated damage, a less virulent infection model was employed. Eight-week-old C3H/HeN mice were immunized and then challenged intraperitoneally with 3×10⁵ blood trypomastigotes of *T. cruzi* K98 clone (DTU Tcl). Although this experiment was setup to study the early chronic phase of infection, parasitemia and body weight were monitored during the acute phase weekly. As expected, all immunized mice presented a reduced parasitemia with respect to the control group in the acute phase of infection, with a significant reduction of area under the curve (Figure 11 A). Blood samples were taken at different time points after infection to determine serum levels of cardiac isoform of the damage-associated enzyme creatine kinase (CK-MB). As shown in Figure 11 B, at 72 dpi (early chronic phase), immunized mice presented a significant decrease of CK-MB levels compared to the control group, which indicates a lower damage on heart tissue during the early chronic phase of the infection. At 100 dpi, electrocardiograms (ECG) were performed to evaluate cardiac electrical activity. The PR interval, a very sensitive parameter to *T. cruzi* infection in murine models, was prolonged in the control group with respect to immunized mice, indicating a higher cardiac risk (Figure 11 C). At 100 dpi mice were euthanized and muscles were removed to analyze the parasite burden, inflammatory foci and fibrosis. The parasite load was assessed by quantitative PCR. Immunized mice showed a clear reduction of parasite loads, as compared with controls, being this reduction significantly more prominent in animals vaccinated with the acetylated Traspain (Figure 11 D). Histological sections of muscles were stained with hematoxylin-eosin or picrosirius red to evaluate inflammatory foci and fibrosis, respectively. In both skeletal and heart muscle, immunized mice showed a significant reduction of the area of inflammatory foci compared to non-vaccinated mice, with animals receiving the acetylated transpain (Tp 10 µg/CDA 50 µg) showing the best trend (Figure 11 E). All immunized mice also presented a significant reduction of the fibrosis area as compared to the control group (Figure 11 F), with a clear trend for a better performance in mice receiving the acetylated Traspain.

### Example 4. Therapeutic effect of Traspain

Considering the protective effect observed in vaccinated animals, it was assessed vaccine performance in a therapeutic setting in an experimental model of bona fide chronic phase of Chagas disease. Specifically, C3H mice infected with the K98 clone of the low-virulence CA-I strain of *T. cruzi* (discrete typing unit I or DTU-I) was used as a model. This particular model was chosen due to its low mortality rate and slow disease progression pattern, allowing the mice to reach the chronic phase of the infection (more than 100 days post-infection), which is necessary to implement our treatment schemes. Despite producing scarce histological alterations of the heart, we have previously demonstrated that cardiac function is significantly altered compared to non-infected mice [Sanchez Alberti, A. et al. Front Immunol., 11, 128 (2020)].

Two-month-old C3H mice were intraperitoneally infected with 2.5 × 10⁵ blood trypomastigotes of *T. cruzi* (CA-I clone K98). At chronic phase (>100 dpi), mice were divided in the following experimental groups (n = 5) receiving different treatment regimens: G1: control mock-treated (saline buffer); G2: Benznidazole (Bz) alone: treatment with 100 mg/Kg/day of Bz from 180 to 185 dpi by oral gavage; G3: Traspain formulated with CDA (TpCDA) as adjuvant as immunotherapy alone (three intranasal doses of Traspain 10 µg + CDA 50 µg at 120, 135 and 150 days post infection); G4: a group combining TpCDA (intranasal with 100 mg/Kg/day of Bz from 180 to 185 dpi by oral gavage; and G5: CDA alone (three intranasal doses of the adjuvant alone at 120, 135 and 150 dpi). A group of 3 mice was left uninfected to have age-matched baseline controls (basal group).

*T. cruzi* infection can affect the electrical activity of the heart leading to electrocardiogram (ECG) alterations such as prolonged PR, QR and QRS intervals. As above mentioned, EGC alterations were detected upon *T. cruzi* murine infection. Particularly, in our study, PR interval was the most robust and sensitive ECG parameter to monitor infection (Figure 12).

Benznidazole-treated groups (alone or in combination with TpCDA) reduced the prolongation of PR, cQT and QRS intervals. However, as shown in Figure 13, the vaccine-linked chemotherapy showed the best performance reaching almost normal values. PR interval in TpCDA/Bz treated group showed significant differences with Control group. Neither immunotherapy alone nor the adjuvant alone were sufficient to improve all the ECG parameters. Thus, the combination treatment was unexpectedly, the most successful intervention to prevent disease onset/progression from the point of view of the electrocardiographic alterations.

At 240 dpi, mice were euthanized, and skeletal and heart muscle were removed to prepare histological sections. Subsequently, they were stained with hematoxylin-eosin or picrosirius red to evaluate inflammatory foci and fibrosis, respectively. As expected, in skeletal muscle numerous inflammatory foci were observed among infected groups. Bz alone has a very modest effect on reducing the area of inflammatory infiltrates. However, the combination with the immunotherapy (TpCDA/Bz group) led to a statistically significantly lower level of inflammation as compared to the Control group. It is important to highlight that the pattern observed in the TpCDA/Bz group cannot be distinguished from the Basal baseline reference group (Figure 14). Neither the vaccine alone nor the adjuvant alone were able to improve the inflammatory foci at the skeletal muscle level in comparison to the Control Group. These results indicate that the combination of immunotherapy with chemotherapy can control the inflammation caused by the parasite in this model of early chronicity.

A similar scenario was observed for assessment of fibrosis. The combination of Bz with the therapeutic vaccine significantly reduced the fibrosis area compared to Control group, showing also significant differences with the therapeutic vaccine alone. As expected, CDA alone was not able to confer any protection showing no significant differences with the Control group. The combination of vaccine and Bz is able to protect the muscles from fibrosis induced by the parasite, although there was a reduction also in the level of fibrosis in the group treated with the vaccine, at the level of the group treated with Bz (Figure 15). We have found that, even when the immunotherapy with acetylated Traspain + CDA reduced the level of fibrosis, the combination of vaccination with Bz chemotherapy was the most effective therapeutic approach. It showed a better performance compared to Tp+CDA or Bz alone, not only by reducing electrophysiology disorders but also by controlling more efficiently the inflammation and fibrosis in chronically infected mice.

The immunotherapy combining vaccination and chemotherapy was administered in combination with other oral therapeutic agent Nifurtimox, or following a therapeutic scheme in which the chemotherapeutic drug is administered before, simultaneously or after the administration of the therapeutic vaccine. The results presented above are representative of these alternative scenarios.

### Example 5. Analysis of the the immune modulatory effects resulting from the combination therapy

At 240 dpi, Traspain-specific cell-mediated immunity was studied through the stimulation of spleen cells with the antigen. Antigen-specific proliferation was studied by the uptake of ³H-thymidine. As shown in Figure 16, only the TpCDA/Bz group showed a significant proliferation compared to the Control group, indicating a stronger cell-mediated immunity compared to the other treatment groups.

The profile of cytokines produced by splenocytes upon the stimulation with Traspain was analyzed by flow cytometry. Polyfunctional cells, defined as those with the ability to produce more than one cytokine at the same time, were also analyzed, since they are responsible for providing superior protection post vaccination against different infectious agents. Frequencies of each defined subset were determined after automatic Boolean combination gates employing FlowJo software. The profile of the immune response triggered was influenced by the adjuvant CDA that displayed a Th1/Th17 bias with a balanced cytokine profile. A response pattern which is desired in order to promote efficient parasite clearance. The groups that received the immunotherapy (Tp-CDA and Tp-CDA/Bz) showed the highest percentage of CD4+ T cells producing IFN-γ, IL-2, TNF-α or IL-17. The bias towards a Th1/Th17 profile displayed by mice of Tp-CDA and Tp-CDA/Bz groups is indicated by the low levels of IL-4 secreting cells and a higher ratio IFN-γ/IL-4 and the production of IL-17, respectively. Vaccine-treated groups also showed a synergistic effect with the highest percentage of polyfunctional T cells producing 2 or more cytokines simultaneously. More interestingly, vaccine-linked chemotherapy induced the highest proportion of polyfunctional T cells, significantly more than the Tp-CDA group (Figure 17).

### Example 6

The experimental data presented in Figures 18 and 19 further demonstrate that even for the expert in the art is not self-evident that the combination of a therapeutic vaccination based on Traspain with a treatment with an antiparasitic drug should necessarily result in a clinical improvement and/or an enhanced anti-parasitic immune response. The form in which both components are administered play a critical role, and the outcome cannot be predicted a priori, but needs to be determined experimentally. As shown in Figure 18 mice that were infected with *T. cruzi* were either vaccinated 3 times with Traspain+CDA and subsequently received a course of Benznidazol or were concurrently treated with Benznidazol and the therapeutic vaccine. The results shown in Figure 19 demonstrate that IFN-γ, TNF-α, IL-2 and IL-17 responses were significantly stronger in animals receiving the sequential than the concurrent treatment scheme. More important, animals receiving the sequential treatment have a very significant reduction in both the inflammatory infiltrates and the electrocardiographic alterations at the end of the experiment respect to those receiving it concurrently, thereby confirming the therapeutic benefit and the fact that the form in which the treatments are administered influence the observed effect.

## Claims

1. A recombinant modified polypeptide comprising a sequence of SEQ ID. No. 1, or that exhibits at least 95% identity to SEQ ID No. 1 or that differs maximally 25 amino acids from the sequence of SEQ ID No. 1 whereby at least one of the cysteines present in SEQ ID. No. 1 is modified by i) mutation including deletion or substitution and/or ii) by chemical modification of the sulfhydryl group present in the cysteine by forming a S-S linkage between the sulfhydryl group and a S-containing moiety.

2. The recombinant modified polypeptide according to claim 1 being an acetylated polypeptide, whereby the acetyl group as linked to an at least one cysteine of SEQ ID. No. 1 with a S-S-linkage between the sulfhydryl group of the cysteine and a S-containing moiety comprising an acetyl group, preferably, wherein at least two cysteines of SEQ ID No. 1 are modified, preferably wherein at least two acetylation of amino acid residue of cysteines of SEQ ID. No. 1 is present.

3. The modified polypeptide according to any one of the preceding claims having a solubility in water of at least 1 mg/ml, preferably at least 2 mg/ml.

4. A method for chemical modification of recombinant polypeptides as defined in any one of claims 1 to 3 comprising the steps of
i) purification of the denaturated polypeptide;
ii) chemical modification, in particular, acetylation of the polypeptide at the sulfhydryl group of at least one cysteine present in the polypeptide of SEQ ID. No. 1 or that exhibits at least 95% identity to SEQ ID. No. 1 or that differs maximally 25 amino acids from the sequence of SEQ ID. No. 1., preferably with a S-containing moiety comprising an acetyl group;
iii) refolding of the recombinant modified polypeptide and purification thereof.

5. The method for acetylation of polypeptides according to claim 4 wherein the acetyl group donor is N-acetyl-cysteine.

6. The recombinant modified polypeptide obtainable with the method according to any one of claims 4 to 5.

7. A composition comprising i) a recombinant modified polypeptide according to any one of claims 1 to 3 or 6 and, ii) an adjuvant and, optionally, iii) an active agent, in particular, a therapeutic agent, like Benznidazol or Nifurtimox.

8. A composition comprising i) a recombinant modified or non-modified polypeptide comprising a sequence of SEQ ID. No. 1 or that exhibits at least 95% identity to SEQ ID. No. 1 or that differs maximally 25 amino acids from the sequence of SEQ ID. No. 1 as defined in any one of claims 1 to 3 or 6 or 7 ii) an adjuvant and iii) an active agent, in particular, a therapeutic agent, like Benznidazol or Nifurtimox.

9. The composition according to claim 7 or 8 wherein the adjuvant is a cyclic dinucleotide, in particular, a cyclic di-AMP and/or comprising further at least one of auxiliaries, stabilizers, preservatives, or mucoadhesives.

10. The composition according to any one of claims 7 to 9 present in at least two separate components wherein the first component comprises the modified, like acetylated polypeptide or the non-modified polypeptide and the adjuvant, and the second component comprises an active agent.

11. The composition according to any one of claims 9 to 13 or the modified, like acetylated polypeptide according to any one of claims 1 to 5 or claim 8 for use in preventive or therapeutic vaccination.

12. A composition for use in therapeutic vaccination comprising
i) a non-modified polypeptide comprising a sequence of SEQ ID. No. 1 or that exhibits at least 95% identity to SEQ ID. No. 1 or that differs maximally 25 amino acids from the sequence of SEQ ID. No. 1;
ii) an adjuvant and optionally,
iii) an active agent, in particular, a therapeutic agent, like a antparasitic drugs including Benznidazol or Nifurtimox.

13. The composition of the modified, in particular, acetylated polypeptide or the non-modified polypeptide for use according to claim 11 or 12 in
i) preventive vaccination for Chagas disease prevention or to prevent the onset or progression of the disease to severe gastro/cardio/neuropathologies in case of infection; or
ii) for therapeutic vaccination for Chagas disease for blocking, delaying or reducing the progression of the gastro/cardio/neuro-pathologies associated to Chagas disease.

14. The composition or the modified, in particular, acetylated polypeptide for use according to claim 11 to 13 wherein the first component comprising the polypeptide and the adjuvant and the second component comprising the active agent are administered simultaneously, separately or subsequently.

15. The composition or the modified, in particular, acetylated polypeptide for use according to any one of claims 11 to 14 wherein the vaccine is administered in combination with another T. cruzi antigen increasing the antigen range of the resulting formulation.

16. The composition or the modified, in particular, acetylated polypeptide for use according to any one of claims 11 to 15 wherein the prophylactic or therapeutic vaccine is administered mucosally, in particular, by intranasal, conjunctival, oral, sublingual, inhalation, or systemically, like intramuscular, subcutaneous, intradermal or transcutaneous by using patches, spray devices, droppers.
